# EUROPEAN PATENT APPLICATION

(11) **EP 4 832 417 A1**
(43) Date of publication of application: **28.10.2026**
(21) Application number: 26172479.3
(22) Date of filing: 15.04.2026
(51) Int. Cl.: H04L 9/32, G16H 40/63, H04L 9/40

(54) **CERTIFICATE MANAGEMENT FOR MEDICAL DEVICES**

(30) Priority: 18.04.2025 US 202563790851 P
(71) Applicant: Welch Allyn, Inc., Skaneateles Falls, NY 13153 (US)
(72) Inventor: BRIGGS, Thomas Henry, Skaneateles Falls, 13153 (US); HENCE, Jerry, Skaneateles Falls, 13153 (US); VANN, John R., Skaneateles Falls, 13153 (US)
(74) Representative: Reddie & Grose LLP

(57) **Abstract**

A medical device imports one or more security certificates from an external source. The medical device associates metadata with the one or more security certificates. The metadata indicates a source or intended use for each of the one or more security certificates. The medical device displays visual objects representing the one or more security certificates within a graphical user interface. The visual objects indicate the source or intended use for each of the one or more security certificates based on the metadata. The medical device deploys the one or more security certificates on a trusted platform module for enabling secure communication with one or more external services based on manipulation of the visual objects within the graphical user interface.

## Description

Medical device authentication and security are critical for safeguarding patient data and ensuring the integrity of healthcare systems. With increasing connectivity of medical devices there is a heightened risk of cyber threats. Robust security measures are essential to protect sensitive patient information from breaches and attacks. Compliance with regulatory standards, like those set by the U.S. Food and Drug Administration (FDA) and Health Insurance Portability and Accountability Act (HIPAA), is vital in ensuring that medical devices are designed with security in mind. Ongoing monitoring, regular updates, and vulnerability assessments are crucial to mitigate risks and maintain the reliability of medical devices throughout their lifecycle.

In general terms, the present disclosure relates to managing security certificates for a medical device. In one possible configuration, one or more security certificates are deployed for enabling secure communication with one or more external services based on manipulation of visual objects displayed within a graphical user interface. Various aspects are described in this disclosure, which include, but are not limited to, the following aspects.

One aspect relates to a medical device comprising: a processing device; and at least one memory device storing software instructions that, when executed by the processing device, cause the processing device to: import one or more security certificates from an external source; associate metadata with the one or more security certificates, the metadata indicating a source or intended use for each of the one or more security certificates; display visual objects representing the one or more security certificates within a graphical user interface, wherein the visual objects indicate the source or intended use for each of the one or more security certificates based on the metadata; and deploy the one or more security certificates on a trusted platform module for enabling secure communication with one or more external services based on manipulation of the visual objects within the graphical user interface.

Another aspect relates to a method of managing digital certificates for a medical device, the method comprising: importing one or more security certificates from an external source; associating metadata with the one or more security certificates, the metadata indicating a source or intended use for each of the one or more security certificates; displaying visual objects representing the one or more security certificates within a graphical user interface, wherein the visual objects indicate the source or intended use for each of the one or more security certificates based on the metadata; and deploying the one or more security certificates on a trusted platform module for enabling secure communication with one or more external services based on manipulation of the visual objects within the graphical user interface.

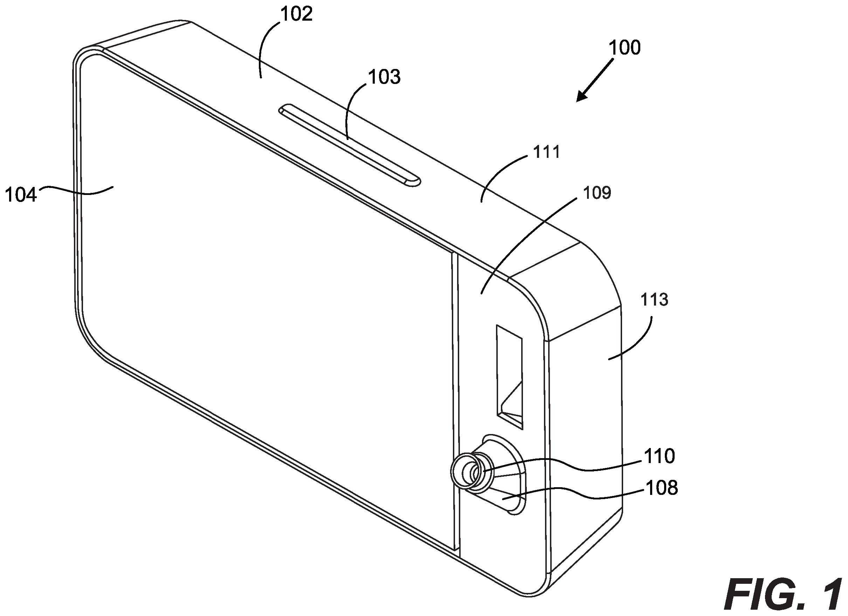

The invention will now be further described by way of example with reference to the accompanying drawings, in which: FIG. 1 is a front isometric view of a medical device for monitoring one or more physiological variables of a patient.
FIG. 2 is a rear isometric view of the medical device of FIG. 1.
FIG. 3 is a front view of the medical device of FIG. 1.
FIG. 4 is a bottom view of the medical device of FIG. 1.
FIG. 5 schematically illustrates an example of the medical device of FIG. 1.
FIG. 6 illustrates an example of a trusted platform module (TPM) that provides secure storage for security certificates installed on the medical device of FIG. 1.
FIG. 7 schematically illustrates an example of a method of managing digital certificates for the medical device of FIG. 1.
FIG. 8 schematically illustrates an example of sub-operations performed in an operation of the method of FIG. 7.
FIGS. 9-16 illustrate examples of graphical user interfaces that can be displayed on a display screen of the medical device of FIG. 1 for certificate management.

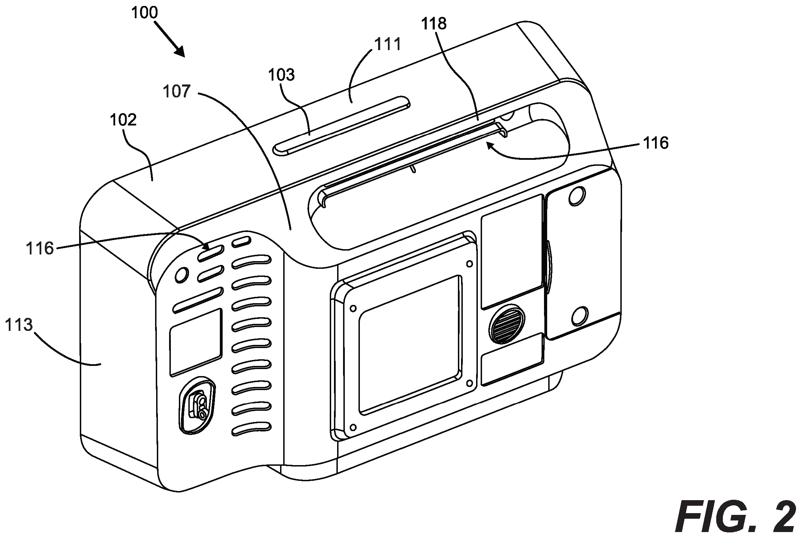

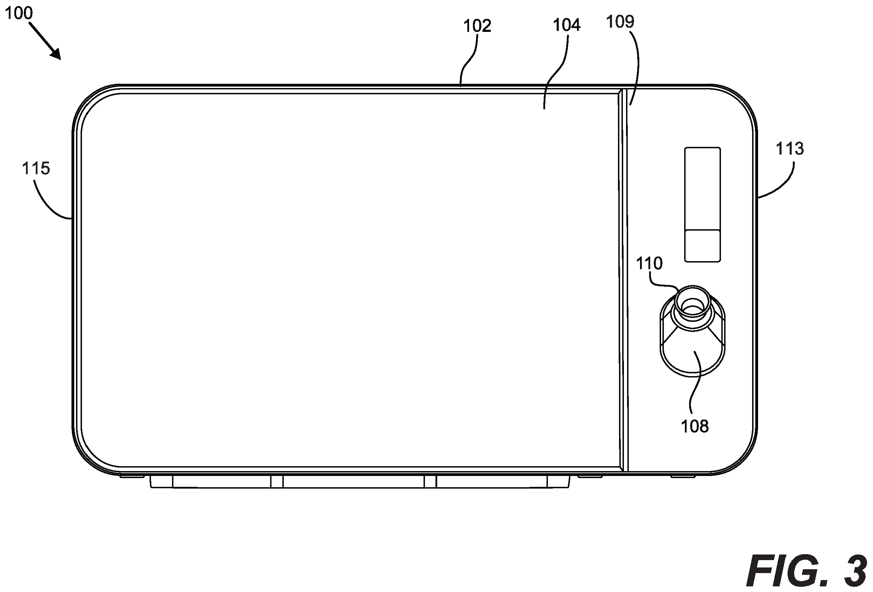

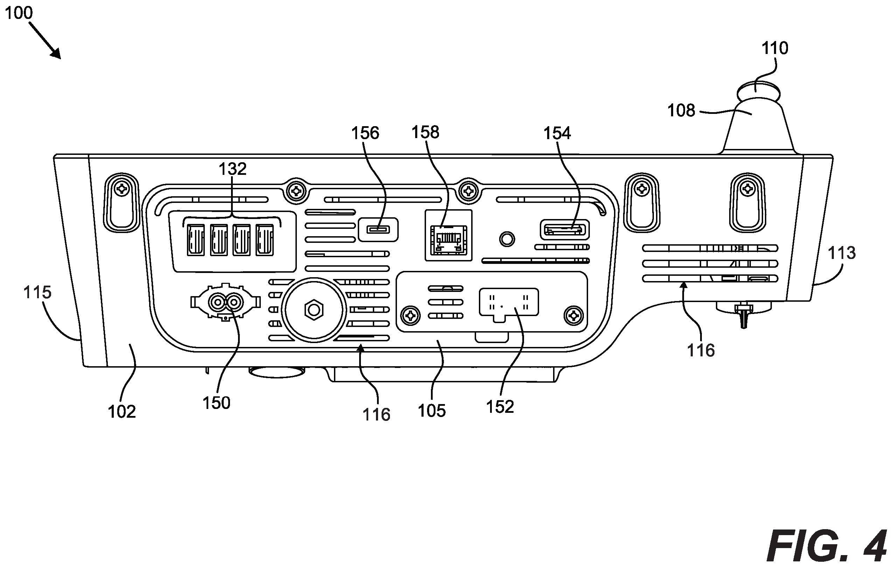

FIG. 1 is a front isometric view of a medical device 100 for monitoring one or more physiological variables of a patient. FIG. 2 is a rear isometric view of the medical device 100. FIG. 3 is a front view of the medical device 100. FIG. 4 is a bottom view of the medical device 100. As shown in FIGS. 1-4, the medical device 100 includes a housing 102 having a bottom surface 105, a rear surface 107, a front surface 109, and a top surface 111 which give the medical device 100 a rectangular prism shape. The medical device 100 is designed for portability by having a handle 118 on the top surface 111 (FIG. 2) that allows the housing 102 to be gripped with one hand for physically moving the medical device 100 from one location to another.

The housing 102 further includes a plurality of vents 116 for dissipating hot air away from internal components of the medical device. The vents 116 are positioned at different areas of the housing 102 such as on the bottom surface 105 (FIG. 4) and the rear surface 107 (FIG. 2).

The display screen 104 is mounted on a front surface 109 of the housing 102. The display screen 104 is a touch sensitive touchscreen that receives tactile inputs from the user of the medical device 100 such as a physician, a nurse, or other trained healthcare professional.

The medical device 100 is designed for use in a clinical setting. For example, the medical device 100 can be used to continuously monitor one or more physiological variables of a patient admitted to a medical facility such as a hospital, a medical clinic, and the like. Additionally, the medical device 100 can operate as a spot monitor for episodic monitoring of one or more physiological variables of a plurality of patients in the medical facility.

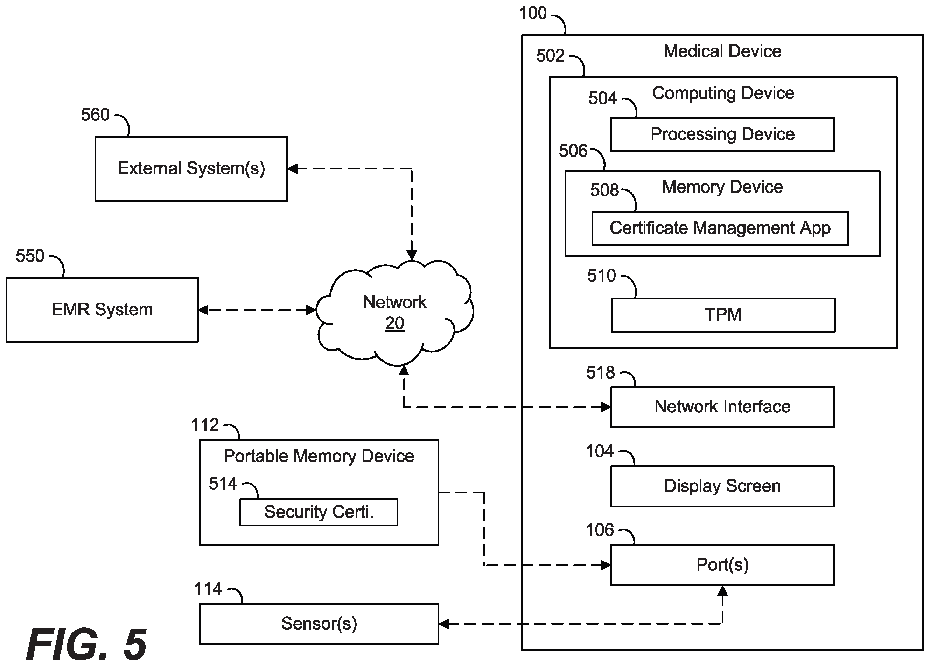

The medical device 100 is configured for connection to one or more sensors 114 (see FIG. 5) that can be used to measure the one or more physiological variables including, without limitation, blood pressure, blood oxygen saturation (SpO2), heart rate, pulse rate, body temperature, and respiration rate. The sensors 114 can include a thermometer module 108 to measure body temperature, a non-invasive blood pressure cuff to measure systolic and diastolic blood pressure, a pulse oximeter to measure blood oxygen saturation and pulse rate, and can include additional types of sensors for measuring additional types of physiological variables.

The sensors 114 can include cables that terminate into connectors that plug into various ports included on the housing 102 of the medical device 100 for transmission of data to the medical device 100. As shown in FIG. 4, the housing 102 includes a first port 150 for connecting the non-invasive blood pressure cuff and a second port 152 for connecting the pulse oximeter. Optionally, the medical device 100 can include a third port 154 for connecting the medical device 100 to a printer. Also, the medical device 100 can include one or more universal serial bus (USB) ports 132, a USB-C port 156, and a registered jack (RJ) interface 158. In some examples, at least some of the sensors 114 wirelessly transmit data to the medical device 100 via a wireless connection established through Wi-Fi, Bluetooth, or another wireless protocol.

In further examples, the housing 102 includes a port for connection of a scanner to the medical device 100. The scanner can be used to scan machine-readable data such as a barcode including linear barcodes, quick-response (QR) codes, and the like that identify a person such as a patient who is being monitored by the medical device 100 or a user of the medical device 100 (e.g., a nurse, a doctor, a caregiver, and the like). The machine-readable data can further identify an object such as medical equipment or a medication being administered to the patient.

In the example shown in FIGS. 1-4, the thermometer module 108 is integrated with the medical device 100. The thermometer module 108 includes a port 110 for housing a handheld probe that can be orally inserted into the mouth of a patient to take a temperature reading. Alternatively, the housing 102 of the medical device 100 can be configured to house a handheld probe that can be inserted into an ear of the patient to take a temperature reading.

The medical device 100 receives data from the sensors 114 for processing. The medical device 100 generates and displays numerical values and/or waveforms based on the data received from the sensors 114 for monitoring the one or more physiological variables of the patient. The medical device 100 displays the numerical values and/or waveforms of the one or more physiological variables on the display screen 104. Also, the medical device 100 generates alarms when the data received from the sensors 114 indicates an alarm condition is detected.

The alarms generated by the medical device 100 can include a visual alarm displayed on the display screen 104 and an audible alarm emitted by a speaker of the medical device 100. An additional visual alarm can be displayed on an illumination unit 103, which is positioned on the top surface 111 of the housing 102 of the medical device 100. The illumination unit 103 allows for distant viewing of critical alarms. The critical alarms generated on the illumination unit 103 can be viewed across a 360 degree field of view around the medical device 100. In some examples, both a visual alarm is displayed on the display screen 104 and a further visual alarm is generated on the illumination unit 103 to indicate a critical status of a detected alarm condition.

The illumination unit 103 can include one or more light-emitting diodes (LEDs) that emit light that is visible through a translucent cover. As an illustrative example, the illumination unit 103 can emit light having a color (e.g., yellow or red) based on a severity of the alarm. Additionally, or alternatively, the illumination unit 103 can emit light flashes based on a predetermined pattern associated with the severity of the alarm (e.g., an increased blinking rate to indicate severe conditions and a decreased blinking rate to indicate less severe conditions).

FIG. 5 schematically illustrates an example of the medical device 100. As shown in FIG. 5, the medical device 100 includes a computing device 502 having at least one processing device 504 and at least one memory device 506 that stores software instructions that, when executed by the at least one processing device 504, cause the at least one processing device 504 to perform the various aspects, functions, and operations described herein.

The at least one processing device 504 is an example of a processing unit such as a central processing unit (CPU). The at least one processing device 504 can include one or more CPUs. In some examples, the at least one processing device 504 includes one or more digital signal processors, field-programmable gate arrays, and/or other types of electronic circuits.

The at least one memory device 506 is an example of a computer-readable data storage device that operates to store data and instructions for execution by the at least one processing device 504. The at least one memory device 506 includes computer-readable media, which includes any media that can be accessed by the at least one processing device 504.

The computer-readable media can include computer-readable storage media and computer-readable communication media. The computer-readable storage media includes volatile and nonvolatile, removable and non-removable media implemented in any device that stores information such as computer-readable instructions, data structures, program modules, or other data. The computer-readable storage media can include random access memory, read only memory, electrically erasable programmable read only memory, flash memory, and other memory technology, including any medium that can be used to store information for access by the at least one processing device 504. The computer-readable storage media is non-transitory.

The computer-readable communication media embodies computer-readable instructions, data structures, program modules or other data in a modulated data signal such as a carrier wave or other transport mechanism and includes any information delivery media. The term "modulated data signal" refers to a signal that has one or more of its characteristics set or changed in such a manner as to encode information in the signal. The computer-readable communication media can include wired media such as a wired network or direct-wired connection, and wireless media such as acoustic, radio frequency, infrared, and other wireless media. Combinations of any of the above are within the scope of computer-readable media.

Security certificates are specialized files that allow devices to securely communicate with external services, systems, and the like. Typically, a security certificate is created and then transferred onto a medium such as a thumb drive for installation onto a device. The installation of the security certificate follows a file-based methodology where the security certificate is imported from the medium for storage on a local memory of the device. The file-based methodology can further include copying the security certificate to a file structure and modifying its configuration, if necessary, which are extra steps for installing the security certificate.

Additionally, it is common to customize a file name to help remember a purpose or intended use for a file. However, once imported and stored onto the local memory of a device, the file name of a security certificate is lost. Instead, the security certificate is associated with a storage area such as slot 1, slot 2...slot n of the local memory. Further, security certificates such as X.509 certificates do not support metadata that can be used to identify the purpose or intended use for the certificates. Instead, a user must remember the purpose or intended use of the security certificate after they have been imported and stored onto the local memory of the device. Further, users who did not install a security certificate will have no way of knowing the purpose or intended use of the security certificate unless they read the internal data of the security certificate to infer its intended use. These limitations make it difficult to manage security certificates, especially in busy environments such as hospitals that have thousands of devices that require periodic updates of the security certificates (e.g., once every 6 or 12 months) for communicating with external services such as EMR systems, service portals, overread services, and the like.

To overcome these challenges, a certificate management application 508 is installed on the at least one memory device 506 to provide a graphical user interface that facilitates security certificate management on the medical device 100. The certificate management application 508 enables deployment of security certificates on the at least one memory device 506 for secure communication with one or more external services based on manipulation of visual objects that represent the security certificates in the graphical user interface.

The certificate management application 508 facilitates installation and assignment of the security certificates by reducing the steps typically needed for deployment of the one or more security certificates. Further, the certificate management application 508 mitigates mistakes and misconfigurations that can occur when installing and assigning the security certificates, and can thereby prevent failed communications with the one or more external services.

The certificate management application 508 performs real-time validation of security certificates during the import process. When a security certificate is identified from an external source such as a portable memory device 112, the certificate management application 508 verifies the certificate's chain of trust by validating the certificate authority's signature using trusted root certificates stored in a dedicated secure storage area of the medical device 100. The certificate management application 508 validates the certificate's expiration dates, cryptographic signatures, and performs format verification to ensure compliance with X.509 standards.

For encrypted certificates, the certificate management application 508 performs a secure decryption process that maintains the security certificate's confidentiality. After successful password entry, the security certificate is decrypted in isolated memory before validation proceeds to ensure sensitive private key data remains protected throughout the import process.

As will be described in greater detail, the certificate management application 508 is configured such that, in response to minimal user interaction via the graphical user interface displayed on the display screen 104, the processing device 504 automatically performs a plurality of certificate management operations without requiring further user input. In particular, upon identification or selection of a security certificate 514 from an external source such as the portable memory device 112, the processing device 504 automatically performs validation of the security certificate 514 including verification of certificate chains, cryptographic signatures, expiration parameters, and format compliance, and, where applicable, performs decryption and subsequent validation in a secure memory environment of the memory device 506. Further, in response to a user action associating a visual object representing the security certificate 514 with a storage slot 512 of a trusted platform module (TPM) 510 or with an external service such as a EMR system 550 or other external systems 560, the processing device 504 automatically executes a multi-step deployment workflow including preparation of the storage slot 512, secure transfer of certificate data to the TPM 510, assignment of a slot identifier, updating of a metadata store 516, and generation of mappings between the security certificate 514 and the external services. During operation of the medical device 100, selection and use of a deployed security certificate 514 for establishing secure communications with an external service are performed automatically based on the metadata stored in the metadata store 516, without requiring user selection of the security certificate 514 at the time of communication. This automated processing reduces manual configuration steps, minimizes user error, and improves the efficiency and reliability of certificate management on the medical device 100.

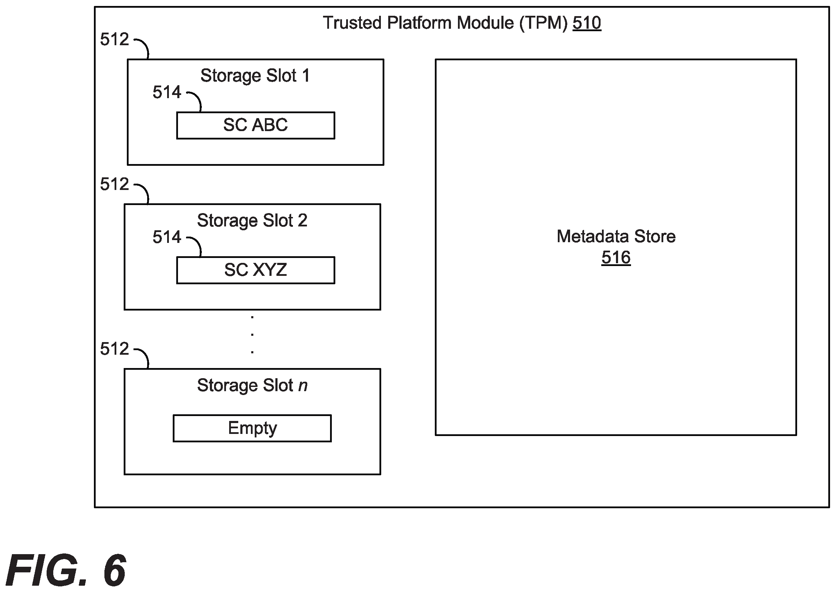

FIG. 6 illustrates an example of the trusted platform module (TPM) 510 that provides a hardware-based secure storage environment for security certificates installed on the medical device 100. The TPM 510 is a specialized chip on the computing device 502 that enhances security by securely storing cryptographic keys used for encryption and decryption, and ensuring the authenticity of the operating system and firmware of the medical device 100. In addition to the cryptographic keys, the TPM 510 can also store the security certificates, passwords, and other sensitive data, protecting them from unauthorized access. The TPM 510 is hardware-based, meaning it operates independently of the operating system of the computing device 502, making the TPM 510 more resistant to software-based attacks. The TPM 510 can be used to encrypt data to ensure that data remains secure even when the medical device 100 is lost or stolen.

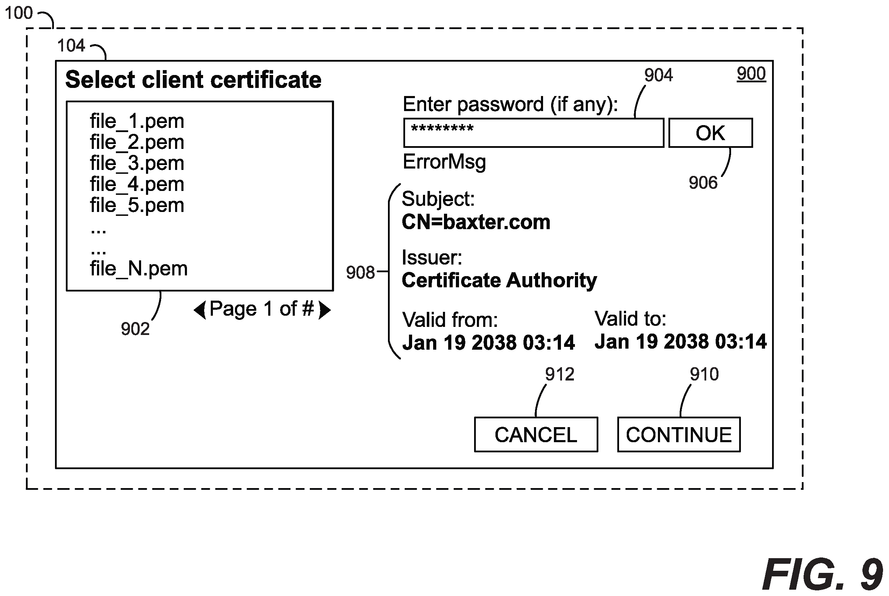

Referring now to FIGS. 5 and 6, the certificate management application 508 interfaces with the TPM 510 to store a security certificate 514 in a storage slot 512 of the TPM 510 when the security certificate 514 is deployed through the graphical user interface generated by the certificate management application 508, as will be explained in more detail with reference to FIGS. 9-16. The TPM 510 maintains isolation between the storage slots 512 and enforces access controls to prevent unauthorized access to the security certificates 514 that are stored on the storage slots 512. At least some of the storage slots 512 in the TPM 510 are reserved for pre-installed system security certificates and cannot have their contents modified through the graphical user interface, while other storage slots of the TPM 510 are available for customer-installed security certificates and can have their contents modified (i.e., a user can replace an existing security certificate with a new security certificate for a particular storage slot).

The certificate management application 508 maintains a metadata store 516 that associates each of the storage slots 512 in the TPM 510 with information about a source or an intended use of a security certificate 514 that has been deployed to a particular storage slot. The metadata store 516 is protected by the TPM 510 but remains distinct from the security certificate data itself, allowing the certificate management application 508 to display security certificate assignments in the graphical user interface without exposing the underlying contents of the security certificates 514. When establishing mutual transport layer security (mTLS) connections, the certificate management application 508 can use the metadata store 516 to identify and/or select an appropriate security certificate from the TPM 510 based on a target service.

As shown in FIG. 5, the medical device 100 includes a network interface 518 that facilitates connection to a network 20 that can include any type of wired or wireless connections or any combinations thereof. The network interface 518 can include wired interfaces and/or wireless interfaces. For example, the network interface 518 can be used to wirelessly connect the medical device 100 to the network 20 such as through Wi-Fi. Alternatively, or additionally, the network interface 518 can be used to connect the medical device 100 to the network 20 using wired connections such as through Ethernet or Universal Serial Bus (USB) cables.

The network interface 518 enables the medical device 100 to communicate via the network 20 with an electronic medical record (EMR) system 550. The EMR system 550 maintains the medical history of a plurality of patients by consolidating health information for each patient into a single document called an electronic medical record (EMR). Each EMR can include patient demographic information, medical diagnoses, treatment plans, medications, test results, physiological variable measurements, and immunization history. The EMR system 550 enables sharing of data among healthcare professionals to improve coordination of care, reduce errors, and enhance patient outcomes. The EMR system 550 also supports clinical decision-making by providing real-time access to comprehensive patient health information. The EMR system 550 can include secure data storage and integration with other healthcare technologies, which streamline administrative tasks and improve overall healthcare efficiency.

The network interface 518 further enables the medical device 100 to communicate via the network 20 with one or more other external systems 560 such as service portals for the medical device, an admission, discharge, and transfer (ADT) system, a laboratory information system (LIS), a nurse call system, and other systems used in a medical facility such as a hospital.

One or more security certificates, also known as public key certificates, digital certificates, or identity certificates are deployed on the TPM 510 to establish the identity of the medical device 100 and to ensure secure communications with external services such as the EMR system 550 and the one or more other external systems 560. The one or more security certificates enable the secure communications with the external services without requiring users of the medical device 100 to enter passwords. Examples of the one or more security certificates include X.509 certificates. Additional examples of the security certificates are contemplated.

The one or more security certificates enable the use of asymmetric cryptography, where a pair of keys are used. A public key (i.e., security certificate) is shared openly and can be freely distributed. The public key is used to encrypt data or verify a digital signature. A private key is kept secret and is used to decrypt the data encrypted with the public key or to create a digital signature. An X.509 certificate is an example of a format for a public key certificate.

An X.509 certificate contains information about the public key, an identity of the owner, and a signature from a trusted entity such as a certificate authority (CA). For example, an X.509 certificate typically includes a subject which is the entity that the certificate is issued to (e.g., a healthcare facility or healthcare network), the public key of the entity, an issuer that issued the certificate (e.g., the CA), a serial number which is a unique identifier for the certificate, a validity period which is a date range indicating when the certificate is valid, a signature algorithm which is the algorithm used by the CA to sign the certificate, and a signature which is a cryptographic signature from the CA proving the certificate's authenticity.

The CA is a trusted third party that vouches for the identity of the entity in the certificate. The CA verifies the identity of the subject and then signs the certificate using its own private key. This creates a chain of trust, meaning that if you trust the CA, you can trust the public key in the certificate. The CA signs the certificate with its private key. This digital signature is included in the certificate and can be verified using the CA's public key. This ensures that the certificate has not been tampered with and was indeed issued by the trusted CA.

As an illustrative example, when the medical device 100 attempts to communicate with the EMR system 550, the medical device 100 sends a security certificate to the EMR system 550. Thereafter, the EMR system 550 checks the security certificate's signature against trusted root certificates. If valid, the EMR system 550 uses the security certificate to initiate a secure communication channel (for example, to exchange session keys) with the medical device 100. This ensures that the communication between the medical device 100 and the EMR system 550 is encrypted and secure. The security certificates provide a secure way to confirm the identity of the medical device 100 and allow for encrypted communication by using a combination of public key cryptography and trusted third parties (certificate authorities (CAs)). The use of the security certificates helps to prevent impersonation, tampering, and man-in-the-middle attacks.

As shown in FIG. 5, a portable memory device 112 can plug into a port 106 of the medical device 100 such as into a USB port 132. In some examples, the portable memory device 112 is a thumb drive, flash drive, pen drive, memory stick, or other portable data storage device. As will be described further below, the portable memory device 112 is an example of an external source that stores one or more security certificates 514 for deployment onto the TPM 510 of the medical device 100 by the certificate management application 508.

Also, the one or more sensors 114 can plug into one or more ports 106 of the medical device 100 such as the first port 150, the second port 152, and the like. Alternatively, the one or more sensors 114 can wirelessly transmit data to the medical device 100 and/or can be integrated with the medical device 100 in accordance with the examples described above.

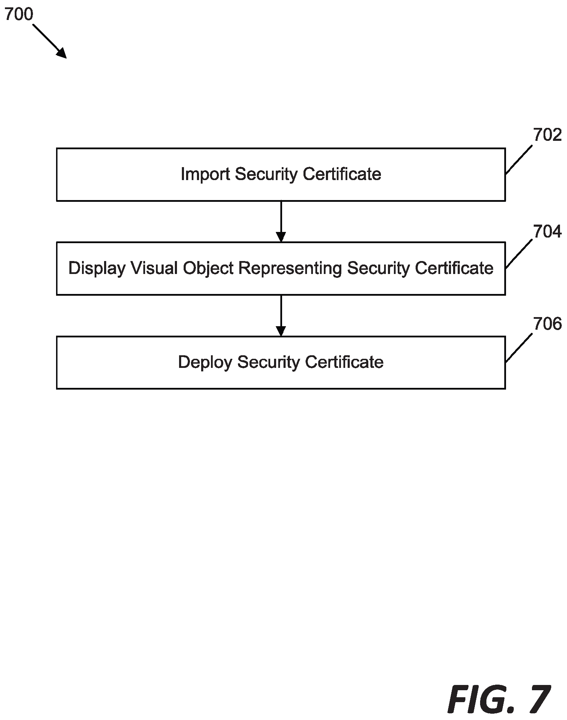

FIG. 7 schematically illustrates an example of a method 700 of managing digital certificates for the medical device 100. The method 700 can be performed by the certificate management application 508 when installed on the medical device 100. The method 700 includes an operation 702 of importing one or more security certificates from an external source such as the portable memory device 112, or a certificate bridge device that is remotely located relative to the medical device 100 and that can communicate with the medical device 100 over the network 20, as described in U.S. Provisional Patent Application No. 63/722,700, filed November 20, 2024, the entire disclosure of which is incorporated by reference herein in its entirety.

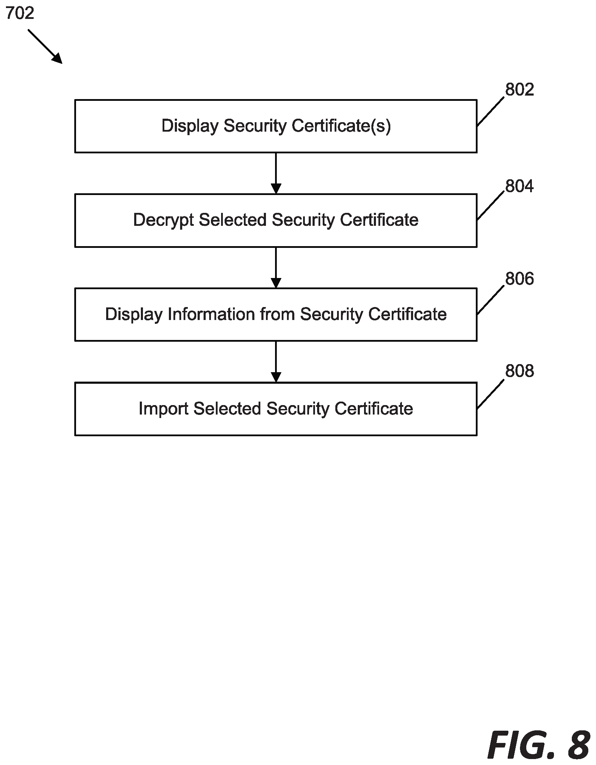

FIG. 8 schematically illustrates an example of sub-operations performed in operation 702 of the method 700. As shown in FIG. 8, operation 702 can include a sub-operation 802 of displaying at least one security certificate identified from the external source. In some examples, operation 702 can include a sub-operation 804 of decrypting a selected security certificate upon entry of a correct password for the selected security certificate. After the selected security certificate is decrypted, or upon selection of the security certificate in examples where no decryption is performed, operation 702 can include a sub-operation 806 of displaying information read from the at least one security certificate. The information can include one or more of a subject of the security certificate, an issuer of the security certificate, a start validity date, and an end validity date. The operation 702 includes a sub-operation 808 of importing the selected security certificate upon selection of an input on the graphical user interface.

FIG. 9 illustrates an example of a graphical user interface 900 that can be displayed on the display screen 104 of the medical device 100 by the certificate management application 508 for execution of sub-operations 802-808 in operation 702 of the method 700. In the example shown in FIG. 9, the graphical user interface 900 includes an area 902 that displays security certificates identified from an external source such as the portable memory device 112 when plugged into a port 106 of the medical device 100 such as into a USB port 132.

In the example shown in FIG. 9, upon selection of a security certificate displayed in in the area 902, a text field 904 is displayed on the graphical user interface 900 for entering a password. In some examples, a virtual keyboard is temporarily displayed on the display screen 104 of the medical device 100 allowing a user to type the password into the text field 904. In examples where the display screen 104 is a touchscreen, a user of the medical device 100 can type the password by touching the display screen 104 where the virtual keyboard is displayed. Alternatively, a physical keyboard hardware can be plugged into one of the ports 106 of the medical device 100 for the user to type the password into the text field 904.

Once the password is typed or otherwise entered into the text field 904, the user can select an Ok input button 906 to enter the password. In examples where the display screen 104 is a touchscreen, the user can simply tap the Ok input button 906 with their finger. Alternatively, when a computer mouse is plugged into one of the ports 106 of the medical device 100, the user can use a pointer or mouse cursor to click on the Ok input button 906.

Upon entry of a correct password in the text field 904, the security certificate selected in the area 902 is decrypted allowing the certificate management application 508 to read information 908 from the security certificate for display on the graphical user interface 900. Alternatively, when the security certificate selected in the area 902 is not encrypted, the information 908 is displayed on the graphical user interface 900 without requiring entry of a correct password in the text field 904. In the example shown in FIG. 9, the information 908 displayed for the security certificate selected in the area 902 includes a subject of the security certificate, an issuer of the security certificate, a start validity date, and an end validity date.

As further shown in FIG. 9, when the information 908 displayed for the security certificate selected in the area 902 is correct, a user can select a continue input button 910 to import the security certificate selected in the area 902 of the graphical user interface 900. Alternatively, the user can select a cancel input button 912 to exit the operation 702 of importing the security certificates from the external source onto the medical device 100.

Referring back to FIG. 7, the method 700 includes an operation 704 of displaying a visual object representing the security certificate imported in operation 702. The visual object is displayed within a graphical user interface displayed on the display screen 104 of the medical device 100. The graphical user interface is generated by the certificate management application 508. The visual object indicates a source or intended use of the security certificate based on metadata appended to the security certificate or otherwise associated with the security certificate.

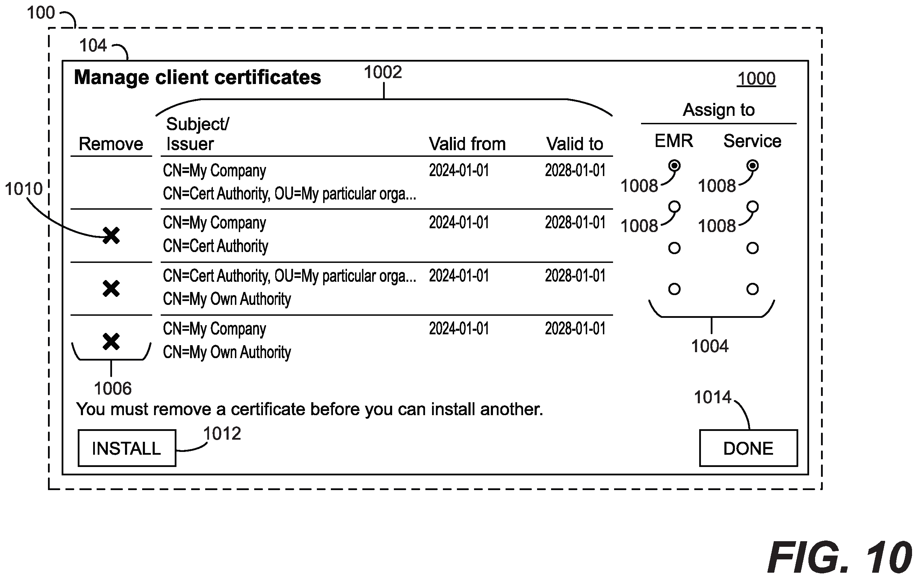

FIG. 10 illustrates an example of a graphical user interface 1000 that can be displayed on the display screen 104 of the medical device 100 for displaying the visual object representing the security certificate in operation 704 of the method 700. The graphical user interface 1000 includes visual objects representing security certificates such as a first column 1002 listing the security certificates. Each horizontal row in the first column 1002 represents a security certificate, and can include information that identifies the security certificate such as subject and/or issuer of the security certificate, a valid from date, and a valid to date.

The visual objects include a second column 1004 having graphical control elements 1008 that allow assignment of a security certificate in a horizontal row of the first column 1002 to the one or more external services. In this illustrative example, the graphical control elements 1008 are radio buttons that can be selected or deselected by touch or by using a pointer or mouse cursor. When selected, the graphical control elements 1008 assign a security certificate in the first column 1002 to one or more external services in the second column 1004.

When deselected, the graphical control elements 1008 unassign the security certificate in the first column 1002 from the one or more external services in the second column 1004. In this example, the second column 1004 includes the EMR system 550 and a service portal as the external services. It is contemplated that the second column 1004 can include additional types of external services, or fewer, as desired.

The visual objects include a third column 1006 having graphical control elements 1010 that when added to the third column 1006 cause a security certificate in the first column 1002 to be deleted from the TPM 510 of the medical device 100. The graphical user interface 1000 has an install icon 1012 that when selected causes installation of a new security certificate on the TPM 510. In this example, the certificate management application 508 requires that an existing security certificate be deleted by the graphical control elements 1010 in the third column 1006 before the new security certificate in the first row can be installed onto the TPM 510.

Referring back to FIG. 7, the method 700 includes an operation 706 of deploying the security certificate on the TPM 510 for enabling secure communication with the one or more external services based on manipulation of the visual objects within the graphical user interface. In FIG. 10, once all selections have been completed on the graphical user interface 1000, selection of a done icon 1014 causes the new security certificate to be deployed on the TPM 510 for use to enable secure communication with the external services selected in the second column 1004 based on manipulation of the visual objects within the graphical user interface 1000 such as selection or deselection of the graphical control elements 1008 in the second column 1004.

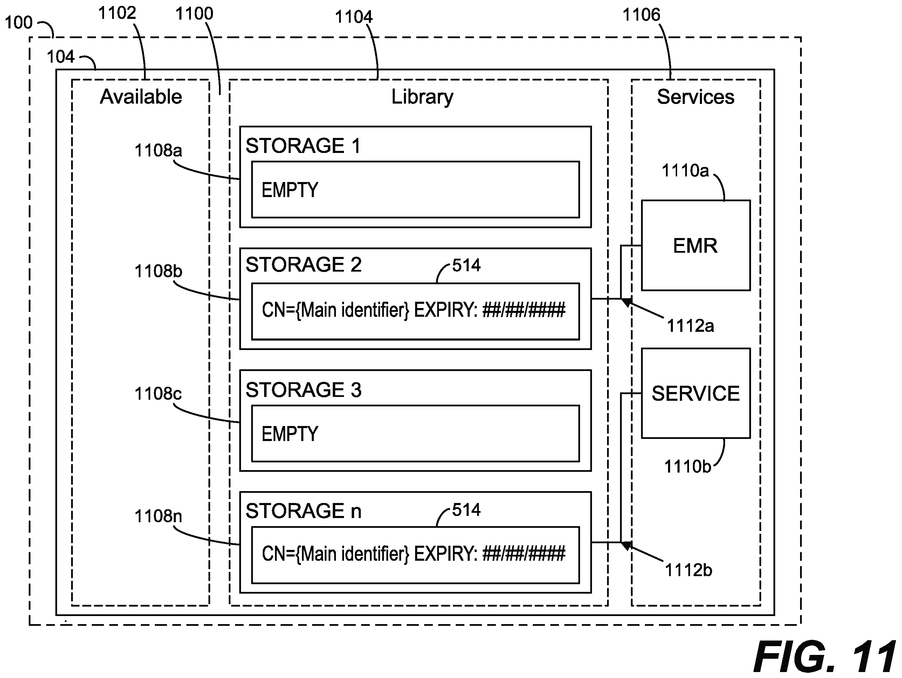

FIG. 11 illustrates another example of a graphical user interface 1100 that can be displayed on the display screen 104 of the medical device 100. Like the graphical user interface 1000 of FIG. 10, the graphical user interface 1100 includes visual objects representing security certificates that can be displayed in operation 704 of the method 700. In this example, the graphical user interface 1100 includes an available security certificate area 1102 where visual objects representing new security certificates are displayed. In this example, the available security certificate area 1102 is empty. This can be due to the graphical user interface 1100 being displayed before the one or more security certificates are imported from the external source such as before the portable memory device 112 is plugged into a port 106 of the medical device 100.

The graphical user interface 1100 further includes a library area 1104 that shows one or more existing security certificates 514 previously imported on the TPM 510. In this example, the library area 1104 includes storage slots 1108 such as a first storage slot 1108a, a second storage slot 1108b, a third storage slot 1108c, and so on. In this example, the first storage slot 1108a and the third storage slot 1108c are empty, while the second storage slot 1108b and a storage slot 1108n each have a security certificate 514 stored therein.

The graphical user interface 1100 further includes a services area 1106 that includes visual objects 1110 representing external services. For example, the services area 1106 is depicted in the example of FIG. 11 as having a first visual object 1110a representing the EMR system 550, and as further having a second visual object 1110b representing a service portal.

The graphical user interface 1100 further displays visual connections 1112 of the one or more security certificates 514 stored in the storage slots 1108 in the library area 1104 to the visual objects 1110 representing the one or more external services in the services area 1106. For example, the graphical user interface 1100 includes a first visual connection 1112a showing that the security certificate 514 stored in the second storage slot 1108b is deployed for secure communication with the EMR system 550 represented by the first visual object 1110a.

A second visual connection 1112b on the graphical user interface 1100 shows the security certificate 514 stored in the storage slot 1108n is deployed to enable secure communication with the service portal represented by the second visual object 1110b in the services area 1106. In the example shown in FIG. 11, the visual connections 1112 include lines to show a mapping between the security certificates 514 stored in the storage slots 1108 to the visual object 1110 representing the external services in the services area 1106.

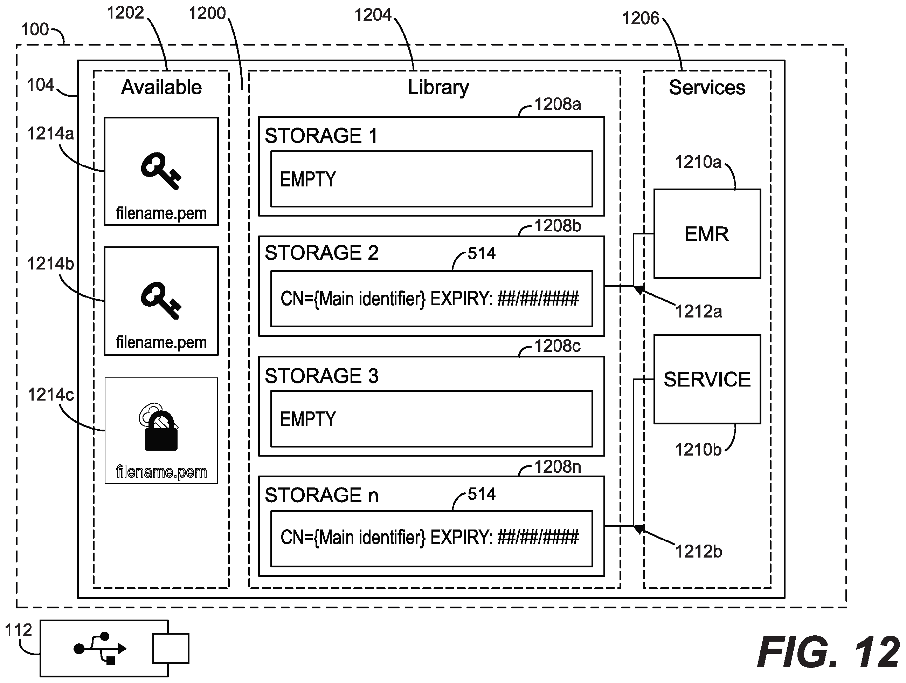

FIG. 12 illustrates another example of a graphical user interface 1200 that can be displayed on the display screen 104 of the medical device 100. The graphical user interface 1200 is similar to the graphical user interface 1100 of FIG. 11 except now the available security certificate area 1202 includes visual objects 1214 representing the security certificates 514 that have been identified from an external source such as the portable memory device 112. The new security certificates 514 represented by the visual objects 1214 in the available security certificate area 1202 are distinct from the existing security certificates 514 previously imported into the storage slots 1208b, 1208n in the library area 1204.

In the example of FIG. 12, a first visual object 1214a representing a first security certificate 514 and a second visual object 1214b representing a second security certificate 514 both identified from the portable memory device 112 are respectively available to be imported and deployed for use with the external services represented by the visual objects 1210 displayed in the services area 1206. A third visual object 1214c representing a third security certificate identified from the portable memory device 112 shows that the third security certificate is not available to be imported and deployed for use with the external services. This can occur when the third security certificate is already deployed in a storage slot 1208 in the library area 1204, or when the third security certificate requires entry of a password to decrypt the security certificate before the security certificate can be imported onto a storage slot 1208 in the library area 1204.

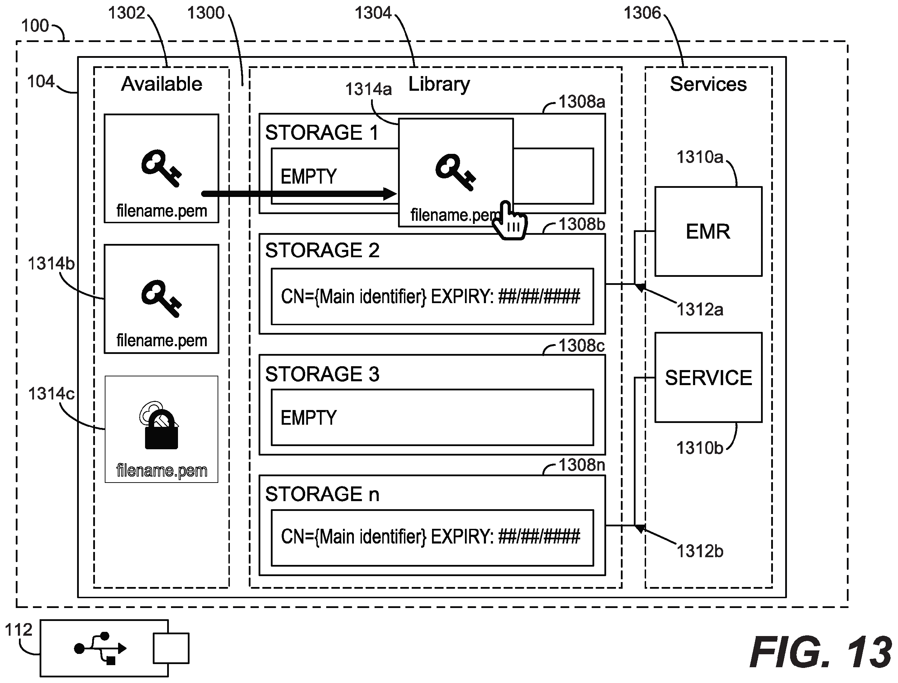

FIG. 13 illustrates another example of a graphical user interface 1300 that can be displayed on the display screen 104 of the medical device 100. The graphical user interface 1300 is similar to the graphical user interface 1200 of FIG. 12 except now the first visual object 1314a is illustrated as being dragged from the available security certificate area 1302 to the first storage slot 1308a in the library area 1304 of the graphical user interface 1300. Accordingly, the security certificates 514 that are represented by the visual objects 1314 in the available security certificate area 1302 can be dragged onto a storage slot 1308 in the library area 1304 via a pointer or mouse cursor, or by touch when the display screen 104 is a touchscreen.

The certificate management application 508 identifies the drag and drop action as an installation of the security certificate onto the TPM 510. As a result, the certificate management application 508 copies the security certificate 514 from the external source such as the portable memory device 112. After the visual object representing the security certificate 514 is dragged and dropped onto the storage slot 1308 in the library area 1304, the certificate management application 508 can perform workflow checks for invalid formats, input/output errors, and provide confirmation when replacing an existing security certificate in a storage slot 1308.

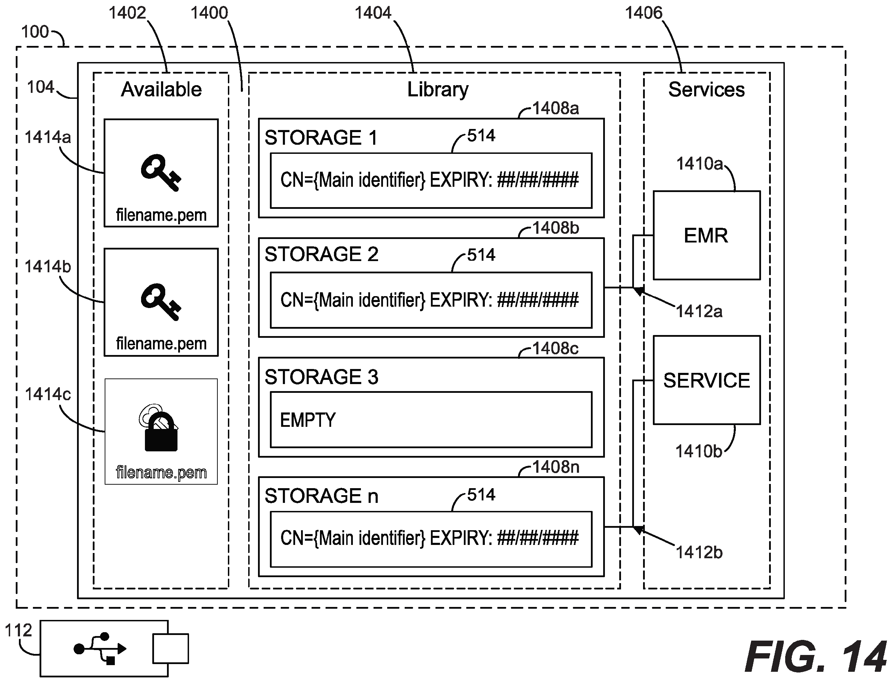

FIG. 14 illustrates another example of a graphical user interface 1400 that can be displayed on the display screen 104 of the medical device 100 after transferring the security certificate 514 represented by the first visual object 1414a from the available security certificate area 1402 to the first storage slot 1408a in the library area 1404. As shown in FIG. 14, after the drag and drop action is complete, the first visual object 1414a returns to the available security certificate area 1402, and the first storage slot 1408a is updated to show that it contains the security certificate 514 represented by the first visual object 1414a.

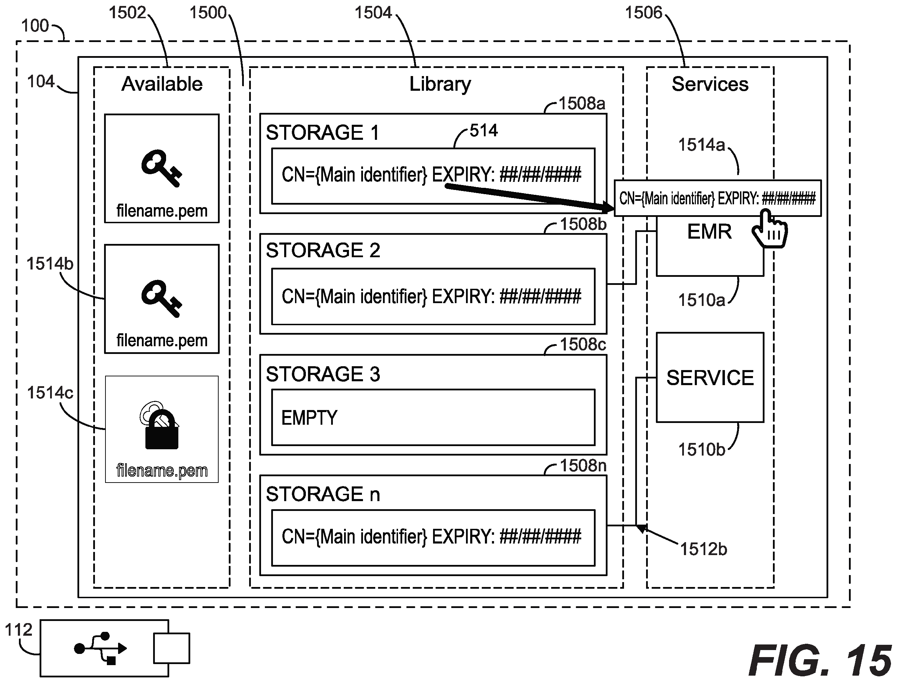

FIG. 15 illustrates another example of a graphical user interface 1500 that can be displayed on the display screen 104 of the medical device 100. The graphical user interface 1500 shows that a virtual object 1514a representing a security certificate 514 stored in the first storage slot 1508a in the library area 1504 is being dragged from the first storage slot 1508a in the library area 1504 to any visual object 1510 representing an external service in the services area 1506. In the example shown in FIG. 15, the virtual object 1514a is being dragged to the first visual object 1510a representing the EMR system 550 to designate the security certificate 514 stored in the first storage slot 1508a for use with the EMR system 550 such as to enable secure communications between the medical device 100 and the EMR system 550.

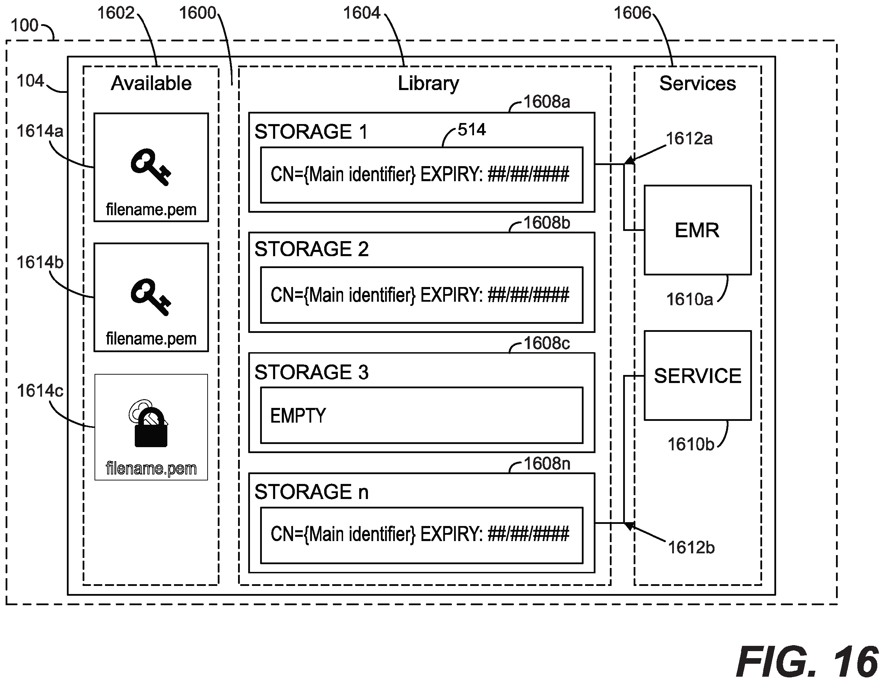

FIG. 16 illustrates another example of a graphical user interface 1600 that can be displayed on the display screen 104 of the medical device 100. The graphical user interface 1600 shows the mapping of the security certificates in the library area 1604 to the external services in the services area 1606 is updated after the drag and drop operation in FIG. 15 is completed. For example, the graphical user interface 1600 is updated to show a visual connection 1612a of the security certificate 514 installed in the first storage slot 1608a to the first visual object 1610a representing the EMR system 550 in the services area 1606. In this example, the visual connection 1612a is a line that shows the security certificate 514 stored in the first storage slot 1608a is connected to the first visual object 1610a representing the EMR system 550.

When a visual object 1610 representing an external service had a previous designation of a security certificate in a storage slot 1608 of the library area 1604, the previous visual connection to the security certificate is deleted. For example, the first visual connection 1112a shown in FIG. 11 that connects the security certificate stored in the second storage slot 1108b to the first visual object 1110a representing the EMR system 550 is replaced in FIG. 16 with the visual connection 1612a connecting the security certificate 514 stored in the first storage slot 1608a to the first visual object 1610a representing the EMR system 550.

By adding the metadata in the metadata store 516 and streamlining the process via the graphical user interfaces shown in FIGS. 9-16, a user can perform an installation or reconfiguration of security certificates without regards to the certificates contents, or the secure infrastructure of the medical device 100. The method 700 follows a more human-like workflow of "I want to use THIS certificate to talk to THIS system". The mechanics of the security certificates are hidden from the user, which simplifies the installation or reconfiguration process.

In view of FIGS. 11-16, the certificate management application 508 implements a secure interface between the graphical drag-and-drop operations and the storage architecture of the TPM 510. When a user drags a visual object representing a security certificate onto a storage slot in the library area of the graphical user interface, the certificate management application 508 initiates a multi-step secure deployment process. First, the certificate management application 508 validates the security certificate as described above, then interfaces with the TPM 510 to prepare a protected storage slot. The TPM 510 enforces hardware-level isolation between slots to maintain security boundaries between security certificates.

During the drag operation, the certificate management application 508 maintains the security certificate data in the TPM 510 while displaying only the metadata-based visual representation to the user. For example, when a drag operation is completed by dropping a visual object onto a valid storage slot in the library area, the certificate management application 508 securely transfers the security certificate data to the TPM 510 through a protected channel. The TPM 510 then generates a unique slot identifier that the certificate management application 508 associates with the visual object representation in the graphical user interface.

The certificate management application 508 implements the drag-and-drop assignment of security certificates to services through a metadata management system that maintains the service associations separately from the security certificate data stored in the TPM 510. When a user drags a security certificate from a storage slot to a service icon in the services area, the certificate management application 508 updates the protected metadata store to create the service assignment mapping while the actual certificate remains securely stored in the designated slot of the TPM 510. This separation allows the intuitive visual interface to manage security certificate assignments without exposing the underlying secure storage architecture.

The visual connections displayed in the graphical user interface 1600, such as lines showing mappings between storage slots in the library area 1604 and service icons in the services area 1606, are generated by the certificate management application 508 based on the protected metadata store. When establishing secure communications with external services, the certificate management application 508 uses these mapped associations to retrieve the appropriate security certificate from the designated memory slot in the TPM 510 through the secure interface. This architecture enables the simplified drag-and-drop interface while maintaining the security of the security certificate data through hardware-based TPM protection.

The certificate management application 508 provides a technical solution to a technical problem in the healthcare field by providing a standardized interface for managing security certificates on medical devices. The standardized interface can be used for managing security certificates across different medical facilities and medical device configurations. Like standardized medical record formats that enable consistent access to patient data, the certificate management application 508 transforms complex X.509 certificate data into standardized visual objects that can be uniformly managed regardless of the certificate source or intended use.

The certificate management application 508 improves the functioning of the medical device 100 by enabling real-time secure communications with external services such as EMR systems and service portals through simplified certificate management while preventing configuration errors that could compromise patient data security or HIPAA compliance. The integration of the certificate management application 508 with the TPM 510 provides hardware-level security protection without exposing technical underpinnings of the security certificates.

Whenever a security certificate is imported from an external source such as the portable memory device 112, the certificate management application 508 performs real-time format standardization and validation. The certificate management application 508 verifies the certificate structure, validates cryptographic signatures, and confirms compatibility with the security architecture of the medical device 100. This standardization ensures certificates from different sources - whether from hospital IT systems, third-party certificate authorities, or device manufacturers - can be consistently managed through a unified visual interface.

The certificate management application 508 maintains a separate metadata store that enables identification of a source or intended use of the security certificates while preserving the integrity of the underlying security certificate data. This allows the certificate management application 508 to present a simplified interface for managing certificate deployments while ensuring the actual certificate data remains protected within the TPM 510. The management of the metadata transforms how the security certificates are deployed by abstracting the technical complexity of mTLS configurations into intuitive visual mappings between security certificates and external services on the display screen 104 of the medical device 100.

For medical devices having a limited display screen size, the certificate management application 508 implements an alternative deployment architecture while maintaining the same security certificate management workflow. The medical devices host an internal hypertext transfer protocol (HTTP) server through the network interface 518. The internal HTTP server makes the graphical user interfaces for certificate management accessible via a standard laptop or computer with a modern web browser connected to the network 20. The internal HTTP server can provide the same graphical user interfaces for security certificate management (see FIGS. 9-16), including the library area showing storage slots on the TPM 510, visual objects representing the security certificates, and mappings that include the visual connections between the security certificates in the library area and the visual objects representing the external services in the services area, but renders these elements in the web browser rather than on the limited device display. This allows healthcare professionals to use the intuitive graphical user interfaces that are disclosed herein for certificate management even when working with medical devices that have constrained display capabilities, while maintaining the secure TPM-based security certificate storage and metadata management architecture on the medical device itself.

In further alternative examples, the certificate management application 508 can operate as software as a service (SaaS) that is accessible through a web browser over the network 20, or over another network such as the Internet, for managing the security certificates on the medical device 100. In such examples, the certificate management application 508 is installed in a secure environment such as a cloud-based server. The SaaS example embodiment can generate the graphical user interfaces of FIGS. 9-16 for managing the security certificates for the medical device 100. Further, the SaaS example embodiment can be used to manage the security certificates for a plurality of medical devices via custom protocols.

Embodiments of the invention can be described with reference to the following numbered clauses, with preferred features laid out in the dependent clauses:
1. A medical device comprising:
   a processing device; and
   at least one memory device storing software instructions that, when executed by the processing device, cause the processing device to:
      import one or more security certificates from an external source;
      associate metadata with the one or more security certificates, the metadata indicating a source or intended use for each of the one or more security certificates;
      display visual objects representing the one or more security certificates within a graphical user interface, wherein the visual objects indicate the source or intended use for each of the one or more security certificates based on the metadata; and
      deploy the one or more security certificates on a trusted platform module for enabling secure communication with one or more external services based on manipulation of the visual objects within the graphical user interface.
2. The medical device of clause 1, wherein the instructions, when executed by the processing device, further cause the processing device to:
   display at least one security certificate identified from the external source;
   upon selection of the at least one security certificate, display information read from the at least one security certificate including one or more of a subject of the security certificate, an issuer of the security certificate, a start validity date, and an end validity date; and
   import the at least one security certificate upon selection of an input.
3. The medical device of clause 2, wherein the instructions, when executed by the processing device, further cause the processing device to:
   decrypt the at least one security certificate upon entry of a correct password prior to displaying the information read from the at least one security certificate.
4. The medical device of either clause 2 or clause 3, further comprising:
   a touchscreen display, wherein the graphical user interface is displayed on the touchscreen display, and wherein the visual objects include graphical control elements that allow assignment of the at least one security certificate to the one or more external services.
5. The medical device of clause 4, wherein the graphical control elements further allow deletion of one or more security certificates previously deployed on the trusted platform module.
6. The medical device of any preceding clause, further comprising:
   a touchscreen display, wherein the graphical user interface is displayed on the touchscreen display, and wherein the visual objects are manipulatable by touching the touchscreen display to drag the visual objects within the graphical user interface.
7. The medical device of clause 6, wherein the instructions, when executed by the processing device, further cause the processing device to:
   display a mapping of one or more security certificates previously deployed on the trusted platform module to the one or more external services.
8. The medical device of clause 7, wherein the instructions, when executed by the processing device, further cause the processing device to:
   display a new security certificate as available for deployment;
   allow the new security certificate to be dragged onto a storage slot of the trusted platform module to import the new security certificate onto the trusted platform module; and
   allow the new security certificate to be dragged to the one or more external services to designate the new security certificate for use with the one or more external services.
9. The medical device of clause 8, wherein the instructions, when executed by the processing device, further cause the processing device to:
   after the new security certificate is dragged to the one or more external services, update the mapping by displaying within the graphical user interface a visual connection of the new security certificate to the one or more external services.
10. The medical device of clause 9, wherein the instructions, when executed by the processing device, further cause the processing device to:
   delete a visual connection representing a designation of the one or more security certificates previously deployed on the trusted platform module.
11. The medical device of any one of clauses 8 to 10, wherein the instructions, when executed by the processing device, further cause the processing device to:
   after the new security certificate is dragged onto the storage slot of the trusted platform module, perform workflow checks for invalid formats, input/output errors, and confirmation when replacing an existing security certificate in the storage slot.
12. A method of managing digital certificates for a medical device, the method comprising:
   importing one or more security certificates from an external source;
   associating metadata with the one or more security certificates, the metadata indicating a source or intended use for each of the one or more security certificates;
   displaying visual objects representing the one or more security certificates within a graphical user interface, wherein the visual objects indicate the source or intended use for each of the one or more security certificates based on the metadata; and
   deploying the one or more security certificates on a trusted platform module for enabling secure communication with one or more external services based on manipulation of the visual objects within the graphical user interface.
13. The method of clause 12, further comprising:
   displaying at least one security certificate identified from the external source;
   upon selection of the at least one security certificate, displaying information read from the at least one security certificate including one or more of a subject of the security certificate, an issuer of the security certificate, a start validity date, and an end validity date; and
   importing the at least one security certificate upon selection of an input.
14. The method of clause 13, further comprising:
   decrypting the at least one security certificate upon entry of a password prior to displaying the information read from the at least one security certificate.
15. The method of any one of clauses 12 to 14, wherein the graphical user interface is displayed on a touchscreen display, wherein the visual objects include graphical control elements that allow assignment of the at least one security certificate to the one or more external services, and wherein the graphical control elements further allow deletion of one or more security certificates previously imported onto the at least one memory device.
16. The method of clause 12, wherein the graphical user interface is displayed on a touchscreen display, and wherein the visual objects are manipulatable by touching the touchscreen display to drag the visual objects within the graphical user interface.
17. The method of clause 16, further comprising:
   displaying a mapping of one or more security certificates previously deployed on the trusted platform module to the one or more external services.
18. The method of clause 17, further comprising:
   displaying a new security certificate as available for deployment;
   allowing the new security certificate to be dragged onto a storage slot of the trusted platform module to import the new security certificate onto the trusted platform module; and
   allowing the new security certificate to be dragged to the one or more external services to designate the new security certificate for use with the one or more external services.
19. The method of clause 18, further comprising:
   updating the mapping by displaying within the graphical user interface a visual connection of the new security certificate to the one or more external services, and deleting a visual connection representing a designation of the one or more security certificates previously deployed on the trusted platform module for use with the one or more external services.
20. The method of clause 18, further comprising:
   performing workflow checks for invalid formats, input/output errors, and confirmation when replacing an existing security certificate with the new security certificate in the storage slot of the trusted platform module.

## Claims

1. A medical device comprising:
a processing device (504); and
at least one memory device (506) storing software instructions that, when executed by the processing device (504), cause the processing device (504) to:
import one or more security certificates (514) from an external source;
associate metadata with the one or more security certificates (514), the metadata indicating a source or intended use for each of the one or more security certificates (514);
display visual objects representing the one or more security certificates (514) within a graphical user interface displayed on a display screen (104), wherein the visual objects indicate the source or intended use for each of the one or more security certificates (514) based on the metadata; and
deploy the one or more security certificates (514) on a trusted platform module (510) for enabling secure communication with one or more external services (550, 560) based on manipulation of the visual objects within the graphical user interface.

2. The medical device of claim 1, wherein the instructions further cause the processing device (504) to:
display at least one security certificate identified from the external source;
upon selection of the at least one security certificate, automatically display information read from the at least one security certificate including one or more of a subject, an issuer, a start validity date, and an end validity date; and
import the at least one security certificate upon selection of an input.

3. The medical device of claim 2, wherein the processing device (504) is configured to decrypt the at least one security certificate upon entry of a correct password prior to displaying the information.

4. The medical device of any of claims 1-3, wherein the graphical user interface is displayed on a touchscreen display (104), and wherein the visual objects include graphical control elements that allow assignment of the one or more security certificates to the one or more external services (550, 560).

5. The medical device of claim 4, wherein the graphical control elements further allow deletion of one or more security certificates previously deployed on the trusted platform module (510).

6. The medical device of any of claims 1-5, wherein the visual objects are manipulatable by touching the touchscreen display (104) to drag the visual objects within the graphical user interface.

7. The medical device of claim 6, wherein the processing device (504) is further configured to:
display a mapping of one or more security certificates previously deployed on the trusted platform module (510) to the one or more external services (550, 560);
display a new security certificate as available for deployment;
allow the new security certificate to be dragged onto a storage slot (512) of the trusted platform module (510); and
allow the new security certificate to be dragged to the one or more external services to designate the new security certificate for use with the one or more external services.

8. The medical device of any of claims 1-8, wherein the processing device (504) is configured such that, in response to a user interaction with the graphical user interface, the processing device (504) automatically performs one or more of:
validation of the one or more security certificates (514), preparation of a storage slot (512) of the trusted platform module (510), secure transfer of the one or more security certificates (514) to the trusted platform module (510), updating of a metadata store (516), and assignment of the one or more security certificates (514) to one or more external services (550, 560), without requiring further user input.

9. A method of managing digital certificates for a medical device (100), the method comprising:
importing one or more security certificates (514) from an external source;
associating metadata with the one or more security certificates (514), the metadata indicating a source or intended use for each of the one or more security certificates (514);
displaying visual objects representing the one or more security certificates (514) within a graphical user interface, wherein the visual objects indicate the source or intended use for each of the one or more security certificates (514) based on the metadata; and
deploying the one or more security certificates (514) on a trusted platform module (510) for enabling secure communication with one or more external services (550, 560) based on manipulation of the visual objects within the graphical user interface.

10. The method of claim 9, further comprising:
displaying at least one security certificate identified from the external source;
upon selection of the at least one security certificate, displaying information including one or more of a subject, an issuer, a start validity date, and an end validity date; and
importing the at least one security certificate upon selection of an input.

11. The method of claim 10, further comprising decrypting the at least one security certificate upon entry of a password prior to displaying the information.

12. The method of any of claims 9-11, wherein the graphical user interface is displayed on a touchscreen display (104), and wherein the visual objects include graphical control elements that allow assignment and deletion of security certificates.

13. The method of any of claims 9-12, further comprising manipulating the visual objects via drag-and-drop operations within the graphical user interface.

14. The method of claim 13, further comprising:
displaying a mapping of one or more security certificates to the one or more external services;
displaying a new security certificate as available;
allowing the new security certificate to be dragged onto a storage slot (512) of the trusted platform module (510); and
allowing the new security certificate to be dragged to the one or more external services.

15. The method of claim 14, further comprising:
in response to manipulation of the visual objects within the graphical user interface, automatically performing, by a processing device (504), one or more of: validation of the one or more security certificates (514), preparation of a storage slot (512) of the trusted platform module (510), secure transfer of the one or more security certificates (514) to the trusted platform module (510), updating of a metadata store (516), assignment of the one or more security certificates (514) to one or more external services (550, 560), and updating of a mapping between the one or more security certificates (514) and the one or more external services (550, 560), without requiring further user input.
